# EUROPEAN PATENT APPLICATION

(11) **EP 2 181 707 A1**
(43) Date of publication of application: **05.05.2010**
(21) Application number: 08075861.8
(22) Date of filing: 04.11.2008
(51) Int. Cl.: A61K 31/18, A61K 31/439

(54) **Combined use of an alpha-adrenergic receptor antagonist and an anti-muscarinic agent**

(71) Applicant: Astellas Ireland Co., Ltd., Dublin 15 (IE)
(72) Inventor: van Charldorp, Karin Juliette, 2350 AC Leiderdorp (NL); Bosman, Brigitte Johanna Fanny, 2350 AC Leiderdorp (NL); Klaver, Monique Maria Alida, 2350 AC Leiderdorp (NL); Garcia Hernandez, Alberto, 2350 AC Leiderdorp (NL); Drogendijk, Tennis Edwin, 2350 AC Leiderdorp (NL)
(74) Representative: Olthoff, Margaretha

(57) **Abstract**

The combined use of (R)-5-(2-{[2-(2-ethoxyphenoxy)ethyl]amino}propyl)-2-methoxybenzene-1-sulfonamide (tamsulosin), or its pharmaceutically acceptable salt, and (1S)-1-phenyl-1,2,3,4-tetrahydroisoquinoline-2-carboxylic acid (3R)-quinuclidin-3-yl ester (solifenacin), or its pharmaceutically acceptable salt, for the preparation of a medicament for the improvement of lower urinary tract symptoms associated with benign prostatic hyperplasia (LUTSBPH) with a substantial storage component is provided.

## Description

The present invention relates to the combined use of the alpha-adrenergic receptor antagonist (R)-5-(2-{[2-(2-ethoxyphenoxy)ethyl]amino}propyl)-2-methoxybenzene-1-sulfonamide (hereinafter referred to as tamsulosin) or a pharmaceutically acceptable salt thereof, and the anti-muscarinic agent (1S)-1-phenyl-1,2,3,4-tetrahydroisoquinoline-2-carboxylic acid (3R)-quinuclidin-3-yl ester (hereinafter referred to as solifenacin) or a pharmaceutically acceptable salt thereof for the preparation of a medicament for the treatment of lower urinary tract symptoms associated with benign prostatic hyperplasia (LUTS/BPH), with a substantial storage component.

### BACKGROUND OF THE INVENTION

Lower urinary tract symptoms (LUTS) associated with benign prostatic hyperplasia (BPH) or LUTS/BPH, previously referred to as symptomatic BPH, is a common condition in men over the age of 50 years. It occurs in approximately 20% of men younger than 65 years and in 40% of men more than 65 years of age (Br. J. Gen. Pract. (1993) 43:318-321e).

LUTS includes storage symptoms, such as frequency, urgency and nocturia and voiding symptoms, such as hesitancy, weak stream, intermittency, incomplete bladder emptying, dribbling and abdominal straining. Although voiding symptoms are more prevalent than storage symptoms, storage symptoms are considered to be the most bothersome. Storage symptoms also interfere to the greatest extent with daily life activities and have a major impact on quality of life (J. Urol. (1997) 157:885-889). BPH refers to a histological diagnosis: a process characterised by stromal and epithelial cell hyperplasia beginning in the periurethral zone of the prostrate. Although nearly all men develop histological BPH, the degree of prostatic enlargement resulting from hyperplasia is highly variable. Its etiology is not fully understood, but both age and male testosterone levels contribute to its progression.

LUTS associated with BPH may be due to obstruction caused by an increased prostatic size induced by an increase in the number of prostatic cells. It may also be due to obstruction caused by an increased contraction of smooth muscle cells in the prostate, urethra and bladder neck (Br. J. Urol. (1975) 47:193-202). However the relationship between prostatic enlargement and LUTS is not very strong (JAMA (1993) 270(7):860-864; Urology (2001) 58(6 Suppl 1):5-16). There is increasing evidence that besides obstruction at the level of the prostrate, other factors such as detrusor disorders, central nervous system disorders, ageing and/or ischemia may contribute to the development of LUTS associated with BPH (Eur. Urol. (2001) 40(Suppl 4):1-4).

The storage symptoms observed in males with LUTS associated with BPH may be due to co-existing detrusor overactivity, as the occurrence of both conditions increases with age, and could be secondary to bladder outlet obstruction. Bladder outlet obstruction could lead to cholinergic denervation of the detrusor and consequent supersensitivity of muscarinic receptors to acetylcholine. Increased bladder outlet resistance may also result in ischemia, increased detrusor collagen content, changes in electrical properties of detrusor smooth muscle cells and reorganisation of the spinal micturition reflex, all of which are associated with the development of detrusor overactivity in animal models (Eur. Urol. (2006) 49:651-659). The prevalence of detrusor overactivity in men with LUTS associated with BPH has been estimated to be between 40 and 70% (J. Urol. (2001) 66:550; Scand. J. Urol. Nephrol. (2001) 35:463). If left untreated, LUTS can worsen and in the long term may eventually lead to (irreversible) bladder dysfunction and an increased risk of serious complications such as acute urinary retention (AUR) (Eur. Urol. (2001) 39:390-399).

Medical treatment is first-line therapy for LUTS associated with BPH. As representative therapeutic drugs currently in use for prostatic hyperplasia, adrenaline α receptor antagonists aimed at ameliorating the functional occlusion are worldwide known and used clinically. The mechanism of action of the adrenaline α receptor antagonists is based on the notion that the receptor involved in the contraction of the prostate and urethral smooth muscle is the adrenaline α receptor. Tamsulosin is an example of such compound, which mainly targets the prostate. As a consequence the strongest symptomatic improvements as measured with the International Prostate Symptom Score (I-PPS) are seen in the voiding symptoms, in particular improvement of weak stream.

On the other hand, the prostate is a target organ of male hormones, androgens, which are intimately involved in the genesis and development of prostatic hyperplasia. As drugs to inhibit such androgens action, chlormadinone acetate and allylestrenol are used in Japan. In Europe and the U.S.A, finasteride and dutasteride, which are drugs that inhibit the enzyme (5α reductase) that converts testosterone to dihydrotestosterone in the prostate, are used for treatment in order to improve LUTS associated with prostatic diminution.

In view of its mechanism of action, a muscarinic receptor antagonist may be effective in improving storage symptoms, but it could antagonize the improving effect on voiding symptoms, since it may inhibit contraction of the bladder at the time of urination. Actually, when improvement of lower urinary tract symptoms associated with prostatic hyperplasia is desired, a muscarinic receptor antagonist should be administered with great caution or it is contra-indicated because it may bring about urinary retention or anuresis that may necessitate catheterization or surgical operation (Br. J. Urol. (1975), 47(2): 193-202; Folia Pharmacologica Japonica (2003) 12: 331). For example, in Japan, an insert to solifenacin succinate, a muscarinic receptor antagonist, states that it is contra-indicated "in patients with anuresis" because "it may inhibit contraction of the bladder at the time of urination and thus deteriorate the symptom", and that "in patients with diseases accompanied with lower urinary tract occlusion (prostatic hyperplasia, and the like.), "it should be administered with great caution because "it may cause anuresis by its anti-cholinergic action". The insert also gives an important basic warning that "In patients with dysuria (lower urinary tract occlusion diseases [prostatic hyperplasia, and the like] or voiding muscle contraction disturbances, and the like), residual urine volume should be measured before administration of this drug and special tests should be considered when necessary. Also, the patients should be kept under careful observation throughout the administration period by periodically confirming absence of aggravation of dysuria."

However, in spite of the fact that a muscarinic receptor antagonist may worsen voiding symptoms due to its own mechanism of action, the combined use of an alpha-adrenergic receptor antagonist and an anti-muscarinic agent for the treatment of LUTS/BPH has been advocated in EP-1123707. A long list of alpha-adrenergic receptor antagonists and anti-muscarinic agents had been disclosed, but the most preferred combinations were doxazosin and darifenacin and 4-amino-6,7-dimethoxy - 2-(5-methanesulfonamido-1,2,3,4-tetrahydroisoquinol-2-yl)-5-(2-pyridyl)quinazoline (also known as UK 338,003) and darifenacin respectively. The efficacy of the combinations was said to be assessed on the basis of the I-PPS-questionnaire. However, any description of the results of a clinical study was missing from the document. J. Urol. (2005) 174: 1334-1338 described the results of a clinical study in male patients, having a history of over 6 months of proven lower urinary tract symptoms associated with prostatic hyperplasia and having at least 1 urgency episode per day and 8 or more micturitions per day, using the combination of doxazosin, an alpha-adrenergic receptor antagonist, and propiverine hydrochloride, a muscarinic receptor antagonist. As shown in Table 3 of this document, doxazosin alone improved the total I-PSS by 7.3 points while the combination of doxazosin and propiverine hydrochloride improved it by 7.4 points. With respect to the storage symptom score, doxazosin alone improved it by 2.9 points whereas the combination of doxazosin and propiverine hydrochloride improved it by 3.8 points. Thus, with respect to the total I-PSS and storage symptom scores, improvement by the combination therapy with the muscarinic receptor antagonist was better than or similar to improvement by the therapy with the alpha-adrenergic receptor antagonist alone. Regarding the voiding score on the other hand, doxazosin alone improved it by 4.5 points whereas the combination of doxazosin and propiverine hydrochloride improved it by only 3.7 points, indicating that the combination with the muscarinic receptor antagonist lowered the improving effect compared with the alpha-adrenergic receptor antagonist alone. Thus, whilst the combination treatment with the alpha-adrenergic receptor antagonist and the muscarinic receptor antagonist further improved storage symptoms as compared to the treatment with the alpha-adrenergic receptor antagonist alone, the combination treatment antagonised the improving effect in voiding symptoms, namely, it exerted a negative effect on the voiding symptoms. This result could be expected. Propiverine however, did not affect the urinary flow rate and no acute urinary retention (AUR) was observed.

A further report on combination therapy using tamsulosin as the alpha-adrenergic receptor antagonist and tolterodine as the muscarinic receptor antagonist as compared to the single drugs and placebo (JAMA (2006) 296(19): 2319) describes the results of a 12 weeks clinical study in men with LUTS, characterized by an I-PPS-score of 12 or higher) and Overactive bladder (OAB), characterized by 8 or more micturitions/day and 3 or more urgency episodes per day. This document demonstrates that the combination therapy with tamsulosin and tolterodine significantly improved the total I-PSS compared to placebo, and that it also significantly improved, urgency, micturition frequency in 24 hours, and nocturnal micturition frequency each of which are the storage symptom indices, compared to placebo. However, as it appears the results for the combination are not significantly better than for the single drugs. No data were shown about the efficacy of the different treatment groups as to voiding symptoms.

The problem to be solved by the present invention therefore was to find a safe and effective treatment to improve the lower urinary tract symptoms associated with BPH and in particular to improve the storage symptoms, that have shown to be more bothersome, without causing a substantial deterioration of the voiding symptoms.

### SUMMARY OF THE INVENTION

The present invention provides the combined use of (R)-5-(2-{[2-(2-ethoxyphenoxy)ethyl]amino}propyl)-2-methoxybenzene-1-sulfonamide (tamsulosin) or its pharmaceutically acceptable salt, and (1S)-1-phenyl-1,2,3,4-tetrahydroisoquinoline-2-carboxylic acid (3R)-quinuclidin-3-yl ester (solifenacin) or its pharmaceutically acceptable salt, for improvement of lower urinary tract symptoms associated with benign prostatic hyperplasia (LUTS/BPH) with a substantial storage component.

### DETAILED DESCRIPTION OF THE INVENTION

After extensive studies the present inventors found that the combined use of (R)-5-(2-{[2-(2-ethoxyphenoxy)ethyl]amino}propyl)-2-methoxybenzene-1-sulfonamide or a pharmaceutically acceptable salt thereof and (1S)-1-phenyl-1,2,3,4-tetrahydroisoquinoline-2-carboxylic acid (3R)-quinuclidin-3-yl ester or a pharmaceutically acceptable salt thereof demonstrated to be of benefit for the preparation of a medicament for improving storage symptoms in male patients having lower urinary tract symptoms associated with prostatic hyperplasia (LUTS/BPH) with substantial storage symptoms, as diagnosed by means of a total I-PPS-score (LUTS/BPH) and number of micturitions/day and urgency episodes/day (storage symptoms) respectively.

The present inventors further surprisingly found that the combined use of in particular tamsulosin hydrochloride and solifenacin succinate showed that the same combination had no or even an deteriorating effect on male patients having lower urinary tract symptoms associated with benign prostatic hyperplasia, without a substantial storage component.

Tamsulosin or its salt is known as an adrenaline α receptor antagonist (US Patent No. 4,703,063; patent document 1) and its chemical structure is shown below. Tamsulosin is also known as YM617.

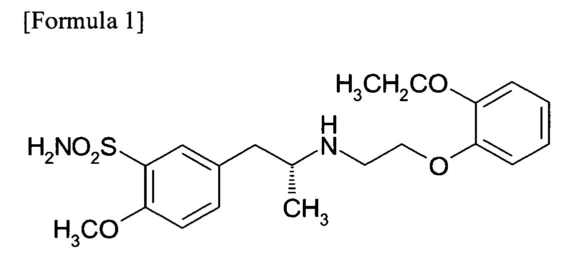

Tamsulosin or its pharmaceutically acceptable salt, an effective ingredient of the pharmaceutical composition of the present invention, is easily available by the methods described, for example, in the aforementioned patent document 1, or by methods obvious to the person skilled in the art, or by their modifications.
Preferably as the first active ingredient (R)-5-(2-{[2-(2-ethoxyphenoxy)ethyl] amino}propyl)-2-methoxybenzene-1-sulfonamide hydrochloride or tamsulosin hydrochloride is used.

This active ingredient can be used in an amount of 0.2 mg or 0.4 mg, dependent on the population to be treated. Normally this active ingredient is included in a modified-release formulation, in the form of coated granules in a capsule (commercially available as OMNIC® or HARNAL® capsules) or in the form of coated matrix tablets (commercially available as OMNIC OCAS® tablets). Alternatively, the active ingredient can be incorporated in a modified-release part of a combination composition.

Solifenacin or its salt is known as a muscarinic receptor antagonist (International patent Publication WO No. 96/20194; patent document 2) and its chemical structure is shown below. Solifenacin is also known as YM905.

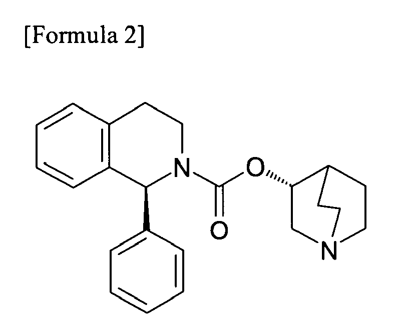

Solifenacin or its pharmaceutically acceptable salt, an effective ingredient of the pharmaceutical composition of the present invention, is easily available by the methods described, for example, in the aforementioned patent document 2, or by methods obvious to the person skilled in the art, or by their modifications.

Preferably, as the second active ingredient, (1S)-1-phenyl-1,2,3,4-tetrahydroisoquinoline-2-carboxylic acid (3R)-quinuclidin-3-yl ester succinate or solifenacin succinate is used.

Dependent on the population to be treated this active ingredient can be used in an amount of 2.5 mg, 5.0 or 10.0 mg or in an amount of 3 mg, 6 mg or 9 mg. The active ingredient is incorporated in an immediate release dosage form, such as a coated tablet (commercially available as VESICARE® tablet), or in an immediate release part of a combination dosage-form.

The "pharmaceutically acceptable salts" in "tamsulosin or its pharmaceutically acceptable salt" and "solifenacin or its pharmaceutically acceptable salt" signify those salts described in the aforementioned Patent Document 1 or the aforementioned Patent Document 2, respectively. Their specific examples are addition salts with inorganic acids such as hydrochloric acid, hydrobromic acid, hydroiodic acid, sulfuric acid, nitric acid, and phosphoric acid, or with organic acids such as formic acid, acetic acid, propionic acid, oxalic acid, malonic acid, succinic acid, fumaric acid, maleic acid, lactic acid, malic acid, mandelic acid, tartaric acid, dibenzoyltartaric acid, ditoluoyltartaric acid, citric acid, methanesulfonic acid, ethanesulfonic acid, benzenesulfonic acid, p-toluenesulfonic acid, aspartic acid, and glutamic acid, and salts with inorganic bases such as sodium, potassium, magnesium, calcium, aluminium, or with organic bases such as methylamine, ethylamine, ethanolamine, lysine, and ornithine, and salts with various amino acids and amino acid derivatives such as acetylleucine, ammonium salts, and others.

Furthermore, the "pharmaceutically acceptable salts" in "tamsulosin or its pharmaceutically acceptable salt" and "solifenacin or its pharmaceutically acceptable salt" may be various hydrates, solvates and polymorphic crystalloids, which are all included as active ingredients for the preparation of the pharmaceutical compositions to be used in accordance with the present invention. The present invention also includes the use of various radioactive or non-radioactive isotope-labeled compounds for the preparation of a pharmaceutical composition to be used according to the invention. The above-mentioned products, containing the first active ingredient, tamsulosin hydrochloride and the second active ingredient, solifenacin succinate, can also form part of a kit.

The present invention provides the use of (R)-5-(2-{[2-(2-ethoxyphenoxy)ethyl]amino}propyl)-2-methoxybenzene-1-sulfonamide or a pharmaceutically acceptable salt thereof for the preparation of a medicament which is simultaneously or sequentially administered with a medicament containing (1S)-1-phenyl-1,2,3,4-tetrahydroisoquinoline-2-carboxylic acid (3R)-quinuclidin-3-yl ester or a pharmaceutically acceptable salt thereof for improving storage symptoms in male patients having lower urinary tract symptoms associated with prostatic hyperplasia (LUTS/BPH) with a substantial storage component.

Further, it provides the use of (1S)-1-phenyl-1,2,3,4-tetrahydroisoquinoline-2-carboxylic acid (3R)-quinuclidin-3-yl ester or a pharmaceutically acceptable salt thereof for the preparation of a medicament which is simultaneously or sequentially administered with a medicament containing (R)-5-(2-{[2-(2-ethoxyphenoxy)ethyl]amino}propyl)-2-methoxybenzene-1-sulfonamide or a pharmaceutically acceptable salt thereof for improving storage symptoms in male patients having lower urinary tract symptoms associated with prostatic hyperplasia (LUTS/BPH) with a substantial storage component.

Also, the present invention provides the use of (R)-5-(2-{[2-(2-ethoxyphenoxy)ethyl]amino}propyl)-2-methoxybenzene-1-sulfonamide or a pharmaceutically acceptable salt thereof and (1S)-1-phenyl-1,2,3,4-tetrahydroisoquinoline-2-carboxylic acid (3R)-quinuclidin-3-yl ester or a pharmaceutically acceptable salt thereof for the preparation of a fixed-dose combination composition for improving storage symptoms in male patients having lower urinary tract symptoms associated with prostatic hyperplasia (LUTS/BPH) with a substantial storage component.

Advantageously the 2 active ingredients are used in fixed dose combination compositions, wherein (R)-5-(2-{[2-(2-ethoxyphenoxy)ethyl]amino}propyl)-2-methoxybenzene-1-sulfonamide hydrochloride or tamsulosin hydrochloride is used in an amount of 0.4 mg and (1S)-1-phenyl-1,2,3,4-tetrahydroisoquinoline-2-carboxylic acid (3R)-quinuclidin-3-yl ester succinate or solifenacin succinate is used in an amount of 3 mg, 6 mg or 9 mg, preferably 6 mg or 9 mg.

The pharmaceutical compositions for use in accordance with the present invention can be prepared by usually employed methods with tamsulosin or its pharmaceutically acceptable salt and solifenacin or its pharmaceutically acceptable salt along with carriers, excipients, or other additives for usual drug formulation. Administration can be performed orally in the forms of tablets, pills, capsules, granules, powders, liquids and the like, or parenterally in the forms of intra-articular, intravenous, or intramuscular injections, suppositories, ophthalmic solutions, ointments, percutaneous liquids, ointments, transdermal patches, transmucosal liquids, transmucosal patches, or inhalants. Oral administration can be cited as a preferred aspect.

As solid compositions for oral administration, tablets, powders, granules, and the like are used. In these solid compositions, the effective ingredient is mixed with at least one of inert excipients, and the like. The composition may follow conventional methods to contain inert additives such as lubricants, disintegrators, stabilizers, or solubilisers. If necessary, tablets and pills may be covered with sugar or gastric- or enteric-coating films.

Liquid compositions for oral administration contain pharmaceutically acceptable emulsifiers, solubilisers, suspensions, syrups, or elixirs, and the like. They also contain generally employed inert diluents such as purified water or ethanol. These liquid compositions may also, in addition to inert diluents, adjuvants such as solubilisers, humidifiers or suspensions, or sweetening agents, flavoring agents, aromatic agents, or antiseptic agents.

Injections for parenteral administration contain aseptic aqueous or non-aqueous solubilisers, suspensions, or emulsifiers. Aqueous solvents include, for example, distilled water for injection or physiological saline. Such compositions may further contain isotonising agents, antiseptic agents, humidifying agents, emulsifying agents, dispersing agents, stabilising agents or solubilising agents. These may be sterilized by filtration through, for example, a bacterium-retaining filter, addition of a sterilizer, or irradiation. These may also be prepared by preparing solid compositions aseptically and then solubilising or suspending them in sterilized water or a sterilized solvent for injection.

Topical preparations include ointments, plasters, creams, jellies, epithems, nebulas, lotions, ophthalmic solutions or ointments, and the like. They contain generally employed ointment bases, lotion bases, aqueous or non-aqueous liquids, suspending agents, emulsifying agents, and the like.

Transmucosal agents such as inhalants or transnasal agents utilize solids, liquids, or semi-liquids, and they can be prepared by conventional methods. For example, known excipients, or further, pH adjusters, antiseptics, surfactants, lubricants, stabilizers, or thickeners may properly be added. Drug administration may be aided by appropriate devices for inhalation or insufflation. For example, by using known devices such as pre-measured inhalation devices or nebulizers, a compound can be administered alone or as a formulated mixture in a powder, or in combination with pharmaceutically acceptable carriers in a solution or suspension. Dry-powder inhalers may be for single or multiple dosage of dry powder or powder-containing capsules, or may be assisted by a pressurized aerosol sprayer utilising appropriate gases such as chlorofluoroalkanes, hydrofluoroalkanes, or carbon dioxide as propellants.

Combination administration in the present invention may be performed by administering two drugs simultaneously, one immediately after the other, or with a desired time interval between them. When administered simultaneously, the two drugs may be administered in a combination composition, that is a single dosage-form in which both effective ingredients are contained, or in two separate formulations, each containing the effective ingredient, which are administered simultaneously.

The combined use of the 2 active ingredients, tamsulosin hydrochloride and solifenacin succinate is particularly useful for improving the symptoms of patients having Lower Urinary Tract Symptoms, associated with Benign Prostatic Hyperplasia (LUTS/BPH) wherein the severity of the lower urinary tract symptoms has been characterised by a total I-PSS score of 13 or higher and wherein the substantial storage symptoms have been characterized as ≥ 8 micturitions/day and ≥ 1, preferably ≥ 2 urgency episodes/day. More particularly the patients have urgency episodes, that are characterized as grade 3 or 4 according to the PPIUS scale.

More particularly the combined use of the first active ingredient and the second active ingredient in accordance with the above has been shown to be very useful for improving the lower urinary tract symptoms associated with BPH in male patients, the symptoms having been characterised by a total score of I-PSS of 13 or higher and wherein the substantial storage symptoms have been characterized as ≥ 8 micturitions/day, ≥ 2 urgency episodes of grade 3 and 4/day and a Qmax of 4-12 mL/s.

The International Prostatic Symptom Scoring (I-PSS) is one of the criteria to evaluate the severity of lower urinary tract symptoms described above, and is used to judge the severity by scoring responses to the following questions:
(1) During the last month, have you had a sensation of residual urine after urinating?
(2) During the last month, have you had to urinate again in less than two hours after you have urinated?
(3) During the last month, have you experienced interrupted urination?
(4) During the last month, have you found it difficult to postpone urination?
(5) During the last month, have you had a weak urinary stream?
(6) During the last month, have you had to strain to urinate?
(7) During the last month, how many times did you typically have to get up to urinate after you went to bed till you got up in the morning?

Out of these questions, the responses to (1) through (6) are scored as 0 if the response was not at all, 1 if less than 1 time in 5, 2 if less than 1 time in 2, 3 if about 1 time in 2, 4 if more than 1 time in 2, and 5 if almost always, and the responses to (7) are scored as 0, 1, 2, 3, 4, and 5 if the response was none, 1 time, 2 times, 3 times, 4 times, and 5 times or more, respectively.
The indices (1), (3), (5), and (6) are those to evaluate voiding symptoms, and (2), (4), and (7) are those to evaluate storage symptoms.

Patients whose total I-PSS scores are 13 or higher are diagnosed as severe enough to be treated in accordance with the method of the present invention. However, a substantial storage component should be present, otherwise the patients will not benefit from the combined use of tamsulosin hydrochloride and solifenacin succinate.

The example further illustrates the invention.

### EXAMPLE

In a single-blind, 2-week placebo run-in period followed by a randomized, double-blind, parallel group, placebo-controlled, 12-week treatment period multi-center dose-ranging study, male patients having LUTS associated with BPH who had voiding symptoms (including incomplete emptying of the bladder, intermittency, weak stream or hesitancy) and storage symptoms (including frequency, urgency or nocturia) for ≥3 months, the severity of their symptoms being characterised by a total International Prostate Symptom Score (I-PSS) of ≥ 1 and a maximum urinary flow rate of ≥4.0 mL/s and ≤15.0 mL/s with a voided volume ≥120 mL, were randomized to 1 of the following 8 treatments:
- placebo,
- monotherapy of 0.4 mg tamsulosin hydrochloride in a modified-release OCAS formulation (commercially available as OMNIC OCAS® tablets),
- monotherapy of 3, 6, or 9 mg respectively of solifenacin succinate in an immediate release tablet formulation or
- combination therapy of 0.4 mg of tamsulosin hydrochloride in a modified-release OCAS formulation (commercially available as OMNIC OCAS® tablets) in combination with 3, 6, or 9 mg respectively of solifenacin succinate in an immediate release tablet formulation.
Throughout the placebo run-in period, subjects took 2 placebo tablets once daily. Throughout the 12-week double-blind treatment period, subjects took 2 tablets once daily (tamsulosin hydrochloride OCAS 0.4 mg or placebo and solifenacin succinate 3, 6 or 9 mg or placebo). Study medication was taken orally in the morning with or without food. Medication was taken with a glass of water and was swallowed whole.

### Efficacy Analyses Results:

Analyses were carried out on subpopulations based on the severity of baseline storage symptoms. The subpopulations investigated were Storage symptoms subgroups 1, 2 and 3, with a daily micturition frequency ≥8 and ≥1, 2 or 3 urgency episodes of grade 3 and 4 per day (PPIUS), respectively. The fourth subgroup was the Limited storage symptoms subgroup, whose baseline symptoms did not meet the criteria for Storage symptoms subgroup 1. Total urgency score, the mean sum of all urgency grades (PPIUS) per day was added as a new parameter.

### Summary of I-PSS data:

Opposite treatment effects were seen between the Limited storage symptoms subgroup and the Storage symptoms subgroups.
• When using higher doses of solifenacin in combination with tamsulosin OCAS alone, a deterioration was seen in the Limited storage symptoms subgroup for total I-PSS score, whereas the Storage symptoms subgroups 1, 2 and 3 showed a non-statistically significant trend to improvement.
• A deterioration in the I-PSS voiding scores was seen for all subgroups, when using combination treatment versus tamsulosin monotherapy. The only statistically significant slope with increasing dosages of solifenacin and in the presence of tamsulosin was in the Limited storage symptoms subgroup (p=0.0006).
• For I-PSS storage scores, all 3 subgroups with Storage symptoms showed a statistically significant improvement in the 3 different combination treatment arms versus monotherapy and in dose response (p=0.0016, p<0.0001, p=0.0006 respectively), while the Limited storage symptoms subgroup showed a not statistically significant deteriorating trend.
• All 3 subgroups with Storage symptoms showed a statistically significant trend to improvement in the I-PSS QoL score (p=0.0094, p=0.0008 and p=0.0106 respectively) with solifenacin-tamsulosin OCAS combination therapy compared to tamsulosin OCAS alone, while the Limited storage symptoms subgroup showed a statistically significant deteriorating trend (see Table 2).

| **Table 2: Parametric modeling results: change from baseline to endpoint in I-PSS scores showing estimated difference between solifenacin - tamsulosin OCAS combination versus tamsulosin OCAS in subpopulations based on severity of baseline storage symptoms (FAS)** | | | | | | | | | | | |
|---|---|---|---|---|---|---|---|---|---|---|---|
| **Parameter** | **Sol dose** | **All subjects** | | **Limited storage Symptoms** | | **Storage Symptoms** | | | | | |
| | | | | | | **Subgroup 1** | | **Subgroup 2** | | **Subgroup 3** | |
| I-PSS Total | n | 704 | | 348 | | 344 | | 282 | | 225 | |
| | 3 mg | 0.14 | | 0.85 | | -0.65 | | -0.81 | | -0.59 | |
| | 6 mg | -0.01 | | 0.80 | | -0.63 | | -1.13 | | -1.44 | |
| | 9 mg | 1.00 | | 2.59 | * 0.0013 | -0.87 | | -1.72 | | -1.56 | |
| | slope | | | | ↓ 0.0026 | | | | | | |
| I-PSS | n | 704 | | 348 | | 344 | | 282 | | 225 | |
| Voiding | 3 mg | 0.49 | | 0.78 | | 0.06 | | 0.1 | | 0.47 | |
| | 6 mg | 0.40 | | 0.57 | | 0.35 | | 0.17 | | -0.01 | |
| | 9 mg | 1.33 | *0.0009 | 2.18 | *0.0002 | 0.25 | | -0.12 | | 0.05 | |
| | slope | | ↓0.0022 | | ↓0.0006 | | | | | | |
| I-PSS | n | 704 | | 348 | | 344 | | 282 | | 225 | |
| Storage | 3 mg | -0.32 | | 0.15 | | -0.7 | *0.0484 | -0.88 | *0.0286 | -0.96 | *0.0389 |
| | 6 mg | -0.41 | | 0.24 | | -0.98 | *0.0071 | -1.28 | *0.0022 | -1.38 | *0.0045 |
| | 9 mg | -0.33 | | 0.44 | | -1.12 | *0.0021 | -1.6 | *0.0001 | -1.59 | *0.0008 |
| | slope | | | | | | ↑0.0016 | | ↑<0.0001 | | ↑0.0006 |
| I-PSS- QoL | n | 703 | | 343 | | 348 | | 281 | | 224 | |
| | 3 mg | -0.13 | | 0.17 | | -0.45 | *0.0171 | -0.5 | *0.0153 | -0.49 | *0.0380 |
| | 6 mg | -0.01 | | 0.31 | | -0.31 | | -0.5 | *0.0189 | -0.38 | |
| | 9 mg | -0.06 | | 0.40 | *0.0277 | -0.58 | *0.0025 | -0.77 | *0.0004 | -0.70 | *0.0043 |
| | slope | | | | ↓0.0196 | | ↑0.0094 | | ↑0.0008 | | ↑0.0106 |

| | | | | | | | | | | | |
|---|---|---|---|---|---|---|---|---|---|---|---|
| Sol dose = solifenacin dosage in mg in combination with tamsulosin OCAS 0.4 mg; Difference on left and significant p-value on right of column; n = total number of subjects in the subgroup with relevant data at any dose of solifenacin; * statistically significant (p<0.05); ↑ statistically significant slope showing improvement; ↓ statistically significant slope showing deterioration (p<0.05) | | | | | | | | | | | |

### Summary of Micturition Diary data:

- Storage symptoms subgroups 2 and 3 showed a statistically significant dose-related improvement on all parameters, when adding solifenacin to tamsulosin OCAS versus tamsulosin OCAS alone. The solifenacin 6 mg - tamsulosin OCAS combination group showed a statistically significant improvement on all parameters, and the solifenacin 9 mg - tamsulosin OCAS combination group on total urgency score, frequency and voided volume (see Table 3.)
- A statistically significant dose-related improvement for urgency episodes of grade 3 and 4 (PPIUS) was seen in Storage symptoms subgroups 2 and 3 (p=0.0128 and p=0.0241, respectively), when using solifenacin - tamsulosin OCAS combination treatment versus tamsulosin alone. There was also a statistically significant improvement for the 6 mg solifenacin - tamsulosin OCAS combination arm versus tamsulosin monotherapy in the 3 subgroups with Storage symptoms.
- For total urgency score there was a statistically significant improvement for the 3 subgroups with Storage symptoms (p=0.0013, p=0.0004 and p= 0.0038, respectively), and a statistically significant improvement for both the 6 and 9 mg solifenacin - tamsulosin OCAS combination arms versus tamsulosin OCAS monotherapy in the 3 subgroups with Storage symptoms.
- The 3 subgroups with Storage symptoms showed a statistically significant dose-related improvement for frequency (p=0.0004, p=0.0003 and p=0.0032, respectively), and all 3 dosages of the solifenacin - tamsulosin OCAS combination arms versus tamsulosin OCAS monotherapy showed a statistically significant improvement.
- When adding solifenacin to tamsulosin OCAS voided volume showed a statistically significant dose-related improvement for all 4 subgroups versus tamsulosin OCAS monotherapy (p<0.0001, p=0.0003, p=0.0042 and p=0.0067, respectively), and also a statistically significant improvement for the 9 mg solifenacin - tamsulosin OCAS combination arm versus tamsulosin OCAS monotherapy in all 4 subgroups.

The positive results in Storage symptoms subgroup 1 were mainly driven by the positive results in Storage symptoms subgroup 2.

| **Table 3: Parametric modeling results: change from baseline to endpoint in micturition diary data showing estimated difference between solifenacin - tamsulosin OCAS combination versus tamsulosin OCAS in subpopulations based on severity of baseline storage symptoms (FAS)** | | | | | | | | | | | |
|---|---|---|---|---|---|---|---|---|---|---|---|
| **Parameter** | **Sol dose** | **All subjects** | | **Limited storage Symptoms** | | **Storage Symptoms** | | | | | |
| | | | | | | **Subgroup 1** | | **Subgroup 2** | | **Subgroup 3** | |
| Urgency | n | 465 | | 124 | | 341 | | 280 | | 224 | |
| 3/4 | 3 mg | -0.1 | | -0.14 | | -0.15 | | -0.06 | | 0.07 | |
| | 6 mg | -0.83 | *0.0075 | -0.24 | | -0.95 | *0.0165 | -1.14 | *0.0138 | -1.47 | *0.0078 |
| | 9 mg | -0.24 | | 0.24 | | -0.47 | | -0.81 | | -0.78 | |
| | slope | | | | | | | | ↑0.0128 | | ↑0.0241 |
| Total | n | 678 | | 335 | | 341 | | 280 | | 224 | |
| Urgency | 3 mg | -1.18 | | -1.15 | | -1.65 | | -1.79 | | -1.57 | |
| score | 6 mg | -1.96 | *0.0055 | -0.53 | | -3.36 | *0.0028 | -3.93 | *0.0026 | -4.57 | *0.0029 |
| | 9 mg | -1.24 | | 0.18 | | -3.2 | *0.0042 | -4.08 | *0.0017 | -3.57 | *0.0166 |
| | slope | | ↑0.0437 | | | | ↑0.0013 | | ↑0.0004 | | ↑0.0038 |
| Frequency | n | 699 | | 347 | | 342 | | 280 | | 224 | |
| | 3 mg | -0.49 | *0.0131 | -0.1 | | -0.88 | *0.0040 | -0.9 | *0.0112 | -1.01 | *0.0129 |
| | 6 mg | -0.66 | *0.0009 | -0.35 | | -0.94 | *0.0028 | -0.96 | *0.0086 | -1.03 | *0.0163 |
| | 9 mg | -0.64 | *0.0012 | -0.2 | | -1.16 | *0.0002 | -1.4 | *0.0001 | -1.3 | *0.0019 |
| | slope | | ↑0.0008 | | | | ↑0.0004 | | ↑0.0003 | | ↑0.0032 |
| Voided | n | 699 | | 347 | | 342 | | 280 | | 224 | |
| volume | 3 mg | 6.3 | | 2.23 | | 10.06 | | 6.46 | | 5.90 | |
| | 6 mg | 18.3 | *<0.0001 | 21.49 | *0.0010 | 14.26 | *0.0341 | 10.18 | | 11.93 | |
| | 9 mg | 24.4 | *<0.0001 | 26.11 | *<0.0001 | 24.45 | *0.0003 | 21.51 | *0.0048 | 21.37 | *0.0097 |
| | slope | | ↑<0.0001 | | ↑<0.0001 | | ↑0.0003 | | ↑0.0042 | | ↑0.0067 |
| Sol dose = solifenacin dosage in mg in combination with tamsulosin OCAS 0.4 mg; Difference on left and significant p-value on right of column; n = total number of subjects in the subgroup with relevant data at any dose of solifenacin; | | | | | | | | | | | |
| * statistically significant (p<0.05); | | | | | | | | | | | |
| ↑ statistically significant slope showing improvement; ↓ statistically significant slope showing deterioration (p<0.05) | | | | | | | | | | | |

## Claims

1. Use of (R)-5-(2-{[2-(2-ethoxyphenoxy)ethyl]amino}propyl)-2-methoxybenzene-1-sulfonamide or a pharmaceutically acceptable salt thereof for the preparation of a medicament which is simultaneously or sequentially administered with a medicament containing (1S)-1-phenyl-1,2,3,4-tetrahydroisoquinoline-2-carboxylic acid (3R)-quinuclidin-3-yl ester or a pharmaceutically acceptable salt thereof for improving storage symptoms in male patients having lower urinary tract symptoms associated with prostatic hyperplasia (LUTS/BPH) with a substantial storage component.

2. The use according to claim 1, wherein (R)-5-(2-{[2-(2-ethoxyphenoxy)ethyl]amino}propyl)-2-methoxybenzene-1-sulfonamide hydrochloride is used as the (R)-5-(2-{[2-(2-ethoxyphenoxy)ethyl]amino}propyl)-2-methoxybenzene-1-sulfonamide or its pharmaceutically acceptable salt.

3. The use according to claim 2, wherein (R)-5-(2-{[2-(2-ethoxyphenoxy)ethyl]amino}propyl)-2-methoxybenzene-1-sulfonamide hydrochloride is used in an amount of 0.2 mg or 0.4 mg.

4. The use according to claim 2 or 3, wherein (R)-5-(2-{[2-(2-ethoxyphenoxy)ethyl]amino}propyl)-2-methoxybenzene-1-sulfonamide hydrochloride is used in a modified-release formulation or in a modified-release part of a combination composition.

5. Use of (1S)-1-phenyl-1,2,3,4-tetrahydroisoquinoline-2-carboxylic acid (3R)-quinuclidin-3-yl ester or a pharmaceutically acceptable salt thereof for the preparation of a medicament which is simultaneously or sequentially administered with a medicament containing (R)-5-(2- {[2-(2-ethoxyphenoxy)ethyl]amino}propyl)-2-methoxybenzene-1-sulfonamide or a pharmaceutically acceptable salt thereof for improving storage symptoms in male patients having lower urinary tract symptoms associated with prostatic hyperplasia (LUTS/BPH) with a substantial storage component.

6. The use according to claim 5, wherein (1S)-1-phenyl-1,2,3,4-tetrahydroisoquinoline-2-carboxylic acid (3R)-quinuclidin-3-yl ester succinate is used as the (1S)-1-phenyl-1,2,3,4-tetrahydroisoquinoline-2-carboxylic acid (3R)-quinuclidin-3-yl ester or its pharmaceutically acceptable salt.

7. The use according to claim 6, wherein (1S)-1-phenyl-1,2,3,4-tetrahydroisoquinoline-2-carboxylic acid (3R)-quinuclidin-3-yl ester succinate is used in an amount of 2.5 mg or 5.0 mg.

8. The use according to claim 6, wherein (1S)-1-phenyl-1,2,3,4-tetrahydroisoquinoline-2-carboxylic acid (3R)-quinuclidin-3-yl ester succinate is used in an amount of 3 mg, 6 mg or 9 mg.

9. The use according to claim 6, 7 or 8, wherein (1S)-1-phenyl-1,2,3,4-tetrahydroisoquinoline-2-carboxylic acid (3R)-quinuclidin-3-yl ester succinate is used in an immediate release formulation or in an immediate release part of a combination composition.

10. Use of (R)-5-(2-{[2-(2-ethoxyphenoxy)ethyl]amino}propyl)-2-methoxybenzene-1-sulfonamide or a pharmaceutically acceptable salt thereof and (1S)-1-phenyl-1,2,3,4-tetrahydroisoquinoline-2-carboxylic acid (3R)-quinuclidin-3-yl ester or a pharmaceutically acceptable salt thereof for the preparation of a fixed-dose combination composition for improving storage symptoms in male patients having lower urinary tract symptoms associated with prostatic hyperplasia (LUTS/BPH) with a substantial storage component.

11. The use according to claim 10, wherein (R)-5-(2-{[2-(2-ethoxyphenoxy)ethyl]amino}propyl)-2-methoxybenzene-1-sulfonamide hydrochloride is used as the (R)-5-(2-{[2-(2-ethoxyphenoxy)ethyl]amino}propyl)-2-methoxybenzene-1-sulfonamide or a pharmaceutically acceptable salt thereof in an amount of 0.4 mg and (1S)-1-phenyl-1,2,3,4-tetrahydroisoquinoline-2-carboxylic acid (3R)-quinuclidin-3-yl ester succinate is used as the (1S)-1-phenyl-1,2,3,4-tetrahydroisoquinoline-2-carboxylic acid (3R)-quinuclidin-3-yl ester or a pharmaceutically acceptable salt in an amount of 6 mg or 9 mg.

12. Use according to any one of the preceding claims wherein the lower urinary tract symptoms have been **characterised by** a total I-PSS score of 13 or higher and wherein the substantial storage component has been **characterized** as ≥ 8 micturitions/day and ≥ 1 urgency episode of PPIUS grade 3 or 4 /day.

13. Use according to claim 12 wherein the substantial storage symptoms have been **characterised** as ≥ 8 micturitions/day and ≥ 2 urgency episodes of grade 3 and 4/day.

14. Use according to claim 12 or 13 wherein the substantial storage symptoms have been **characterized** as ≥ 8 micturitions/day, ≥ 2 urgency episodes of grade 3 and 4/day and a Qmax of 4-12 mL/s.
